# FASCICULE DE BREVET EUROPEEN

(11) **EP 2 528 521 B1**
(45) Date de publication et mention de la délivrance du brevet: **02.03.2016**
(21) Numéro de dépôt: 11704665.6
(22) Date de dépôt: 21.01.2011
(51) Int. Cl.: A61B 17/68

(54) **DISPOSITIF D'OSTEOSYNTHESE DE LA PAROI THORACIQUE**
VORRICHTUNG ZUR OSTEOSYNTHESE DER BRUSTKORBWAND
DEVICE FOR OSTEOSYNTHESIS OF THE THORACIC WALL

(30) Priorité: 26.01.2010 FR 1000275
(43) Date de publication de la demande: 05.12.2012
(73) Titulaire: Université Jean-Monnet, 42023 Saint Etienne Cedex (FR); Choux, Christian, 42600 Savigneux (FR)
(72) Inventeur: CHOUX, Christian, F-42600 Savigneux (FR); TIFFET, Olivier, F-42170 Saint Just-Saint Rambert (FR)
(74) Mandataire: Perrier, Jean-Pierre
(86) Numéro de dépôt international: PCT/FR2011/050121
(87) Numéro de publication internationale: WO 2011/092417

(56) Documents cités:
- FR-A1- 2 353 274
- FR-A5- 2 211 851
- JP-A- 4 156 840

## Description

Ce dispositif d'ostéosynthèse est destiné, après une intervention chirurgicale, à compenser la résection de côtes thoraciques pour éviter l'apparition de déformations de la paroi thoracique, stabiliser celle ci et donc permettre de meilleures fonctions respiratoires, tout en n'altérant pas l'aspect esthétique du thorax.

Actuellement, la réparation thoracique, également appelée reconstruction, est assurée en utilisant les équipement et implants prévus pour traiter les fractures costales suite à un traumatisme grave, et voir même les attelles de maintien des réparations osseuses.

Les implants montrés dans les documents RU2145814, RU2166292, DE3808937, US2436303 et US2005/0216011, mettent en oeuvre des attelles ou barres transversales rigides qui sont disposées devant la paroi thoracique et liées à elle par des moyens d'accrochage divers, et par exemple par des vis vissées dans l'os ou par des griffes agrafées sur l'os. Dans leur application à la reconstruction de la cage thoracique ces implants sont difficiles à poser, forment sur l'avant du thorax des saillies inesthétiques et, surtout, peuvent se désolidariser de la côte avec le risque de migrer et d'entraîner des perforations des organes du voisinnage.

L'implant décrit dans le document US2008/0082101, bien que plus adapté à la reconstruction de la paroi thoracique, est composé d'une attelle rectiligne sur les extrémités de laquelle sont fixées, par des boulons ou vis, des griffes elles mêmes pincées sur les extrémités des côtes résséquées à relier. La rigidité des attelles et des griffes de fixation ne permet pas de les employer dans tous les cas de reconstruction costale et exige pour leur adaptation dimensionnelle et pour leur conformation d'avoir recours à un ancilaire qui, même s'il n'est pas complexe, intervient sur le coût fiinal de la reconstruction.

Par ailleurs, la fixation des implants par des griffes serrées par des pinces sur la côte, blesse cette dernière et présente l'inconvénient de concentrer sur les points de griffage les contraintes transmises à l'implant, en augmentant ainsi ces blessures et la douleur du patient.

Le document JP4156840 décrit un implant composé de deux nappes textiles en fibres polyester liées par des coutures longitudinales l'une à l'autre et autour des extrémités costales délimitant la zone à reconstruire. La gaine tubulaire ainsi réalisée est garnie par un mélange de matériaux liés par une résine. Bien que chaque extrémité de la gaine soit parfaitement adaptée par construction à la forme et aux dimensions de l'extrémité de côte sur laquelle elle est formée, la liaison avec la côte est insuffisante et n'est pas de nature à transmettre à cette côte les contraintes qu'elle reçoit. Il en résulte que l"implant ainsi réalisé est un moyen de garnissage de l'espace entre les deux parties d'une côte réséquée mais ne peut pas être considéré comme un outil de reconstruction remplaçant le tronçon de côte résséqué et transmettant à chaque partie de la côte les efforts et contraintes perçus par l'autre partie.

L'invention à pour objet de remédier à ces inconvénients en fournissant un dispositif d'ostéosynthèse, spécialement adapté à la reconstruction thoracique, facile à mettre en oeuvre, n'exerçant pas de contraintes localisées et traumatisantes sur les côtes, tant à la pose qu'en utilisation, et dont la réalisation finale n'affecte en rien l'esthétisme de la paroi thoraciqiue.

Comme dans l'état de la technique exposé ci dessus, le dispositif selon l'invention est composé d'une gaine tubulaire textile implantable dans le corps humain, apte à contenir un matériau de remplissage et dont les extrémités envelopent les extrémités de deux parties d'une même côte réséquée.

Selon l'invention, la gaine :
- d'une part, contient une tresse, dont les fils, métalliques ou polymériques, acceptent des déplacements relatifs les uns par rapports aux autres pour modifier localement le diamètre et la conformation de la tresse, afin que non seulement elle enveloppe mais aussi enserre l'extrémité d'une partie de côte,
- et, d'autre part, est solidaire d'un embout d'injection d'un matériau biocompatible, assurant le remplissage de la gaine, le garnissage de la tresse et la liaison par collage de la gaine et de la tresse avec les extrémités de la côte.

Grace à cette structure, la pose de l'implant consiste d'abord à adapter la longueur de l'ensemble gaine et tresse à la largeur de l'intervalle entre les bords en vis à vis d'une côte, majorée des longueurs de recouvrement des extrémités, puis à retrousser les extrémités de la gaine sur celles de la tresse pour dégager ces dernières, et à engager chacune des extrémités de la tresse sur les parties en vis à vis de la côte à reconstruire. L'adaptation du diamètre de la tresse au diamètre réel de chaque côte s'effectue en exerçant des efforts longitudinaux opposés sur la tresse, pour augmenter son diamètre ou pour le diminuer. De même, la forme de la section transversale de la tresse est adaptée à celle de l'os, puis il est procédé à la fixation de la tresse sur l'os en maintenant manuellement son extrémité entourant l'os tout en la tirant au delà de lui pour obtenir, par la rétraction diamétrale de la tresse, son serrage sur l'os. Eventuellement, la liaison de la tresse avec chaque partie de côte est complétée par suturage, puis la gaine, jusqu'alors retroussée, est tirée sur chacune des extrémités de la tresse et au delà de ces extrémités, puis fixée à la côte par des sutures ou des agrafes.

La pose de l'implant se termine par l'injection d'un matériau de remplissage dans le cavité interne de la gaine. Pendant le durcissement du matériau de remplisage, le chirurgien peut conformer l'implant afin qu'il reconstitue au mieux la paroi thoracique.

Il apparait déjà que par rapport aux implants comportants des composants métaliques rigides, la mise en place du dispositif selon l'invention n'entraîne aucune blessure des côtes par griffures, conduit à une fixation sûre et efficace dont les contraintes de serrage sont réparties sur tout le pourtour de chaque extrémité de côte et ne sont pas traumatisantes. De plus, la malléabilité des composants permet au chirurgien, pendant le durcissement du matériau de remplissage et sans aucun outillage, de donner à l'implant la conformation qui convient le mieux à la reconstitution de la paroi, tant dans sa fonctionnalité que dans son aspect esthétique.

Enfin, le matériau injecté assure non seulement le remplissage de la gaine mais aussi sa liaison avec les parties de la côte et avec la tresse qui le renforce à la façon d'une armature.

Dès que le matériau injecté est devenu dur et ne risque plus de fluer hors de la gaine, l'embout est sectionné et retiré.

Dans une forme d'exécution de l'invention, la gaine tubulaire textile présente une porosité laissant échapper l'air lors de la coulée du matériau de remplissage mais s'opposant à l'échappement de ce matériau.

Cela permet d'obtenir un remplissage homogène, sans poche de gaz.

Avantageusement, la tresse, la gaine tubulaire et l'embout d'injection sont réalisés dans des matériaux sécables et avec des dimensions permettant de les sectionner avec un ciseau.

Cet aménagement qui réduit l'outillage à une seule paire de ciseaux et supprime tout recours à des pinces coupantes, simplifie l'ajustement de la longueur des composants du corps tubulaire, améliore la précision et réduit le temps de pose.

D'autre caractéristiques et avantages ressortiront de la description qui suit, en référence au dessin schématique annexé, dans lequel :
Figure 1 est une vue de coté en élévation d'une forme d'exécution du corps de l'implant avec coupe partielle de ses extrémités ;
Figure 2 est une vue de coté en élévation d'une cage thoracique humaine montrant quelques unes des zones latérales pouvant être reconstruites ;
Figures 3 à 7 sont des vues partielles en élévation et coupes partielles de l'implant dans différentes phases de la reconstruction d'une côte latérale ;
Figure 8 est une vue de face avec coupes partielles d'une cage thoracique ayant subi une résection du sternum et de trois côtes, quand elle est en cours de reconstruction frontale, et montrant en allant du bas vers le haut, en C6 la mise en place de l'implant, en C5 l'implant après son remplissage et en C4 l'implant terminé et en coupe ;

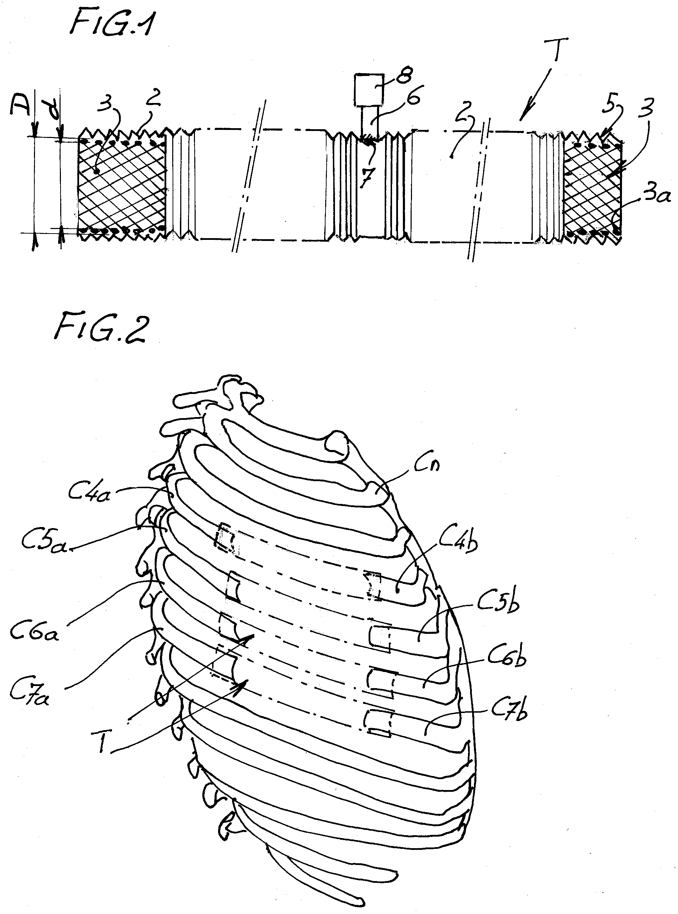

Comme le montre la figure 1, le dispositif selon l'invention est constitué par un corps tubulaire T lui même composé d'une gaine textile extérieure 2 et d'une tresse intérieure 3.

La gaine textile 2 est obtenue par tissage ou tricotage de fils en matériau biocompatible et implantable dans le corps humain et par exemple en polyesters, polytétrafluoréthylène, polyéthylène, propylène...

L'armure du tissu ou la structure du tricot est définie de manière que la gaine 2 soit poreuse à l'air et aux gaz, mais non au matériau de remplissage 4, défini plus loin.

Le diamètre intérieur D de la gaine 2 est compris entre 8 et 14 millimètres, valeurs correspondants aux dimensions les plus courantes d'une côte humaine. Il peut être plus petit pour une application aux animaux.

De préférence, la paroi de la gaine 2 est gaufrée ou annelée par des annelures déformables 5, facilitant l'ajustement de sa conformation et de sa longueur au cours de sa mise en place. Aux figures, ces annelures ont une section triangulaire, mais elles peuvent présenter tout autre section, par exemple, être en arc de cercle ou trapézoïdale.

Suivant une caractéristique de l'invention, la gaine 2 est équipée d'un embout d'injection 6 qui est constitué par un corps tubulaire, textile ou non, en matériau biocompatible. L'une des extrémités de cet embout est liée à la gaine 2, par couture ou soudure 7, tandis que son extrémité libre est munie, ou non, de moyens de connexion 8, permettant son raccordement à un distributeur de matériau de remplissage, et par exemple à l'embout d'une seringue.

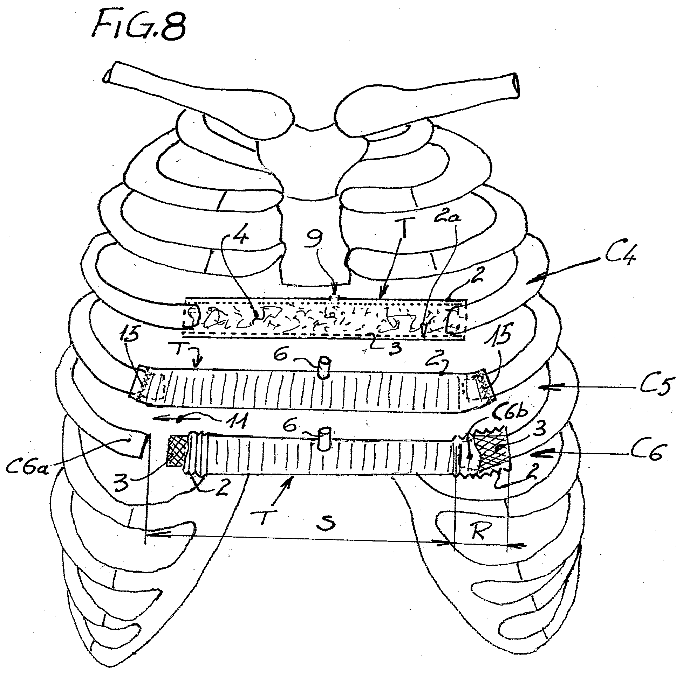

L'embout 6 à une longueur comprise entre 3 et 10 millimètres, est disposé de manière à saillir vers l'extérieur, radialement ou non, et communique avec l'intérieur de la gaine 2 par un trou 9, représenté en C et en haut de la figure 8.

La tresse 3 est composée de fils 3a en matériau biocompatible et implantable dans le corps humain, et par exemple en matériau métallique ou en matière synthétique ou polymérique. Un fil non magnétique est préféré pour éviter de dégrader les résultats d'imagerie médicale, par exemple par Résonnance Magnétique Nucléaire, réalisée lors du suivi postopératoire.

Dans une forme de réalisation, la tresse est composée de fils métalliques tressés avec une angulation comprise entre 30 et 60 degrés et avec la possibilité de se déplacer relativement entre eux pour faire varier le diamètre nominal de la tresse, en plus ou en moins, dans une plage de valeurs. La dimension diamétrale des fils est comprise entre 0,1 et 0,3 millimètres et la tresse est formée par entrecroisement de 16 à 48 brins, en fonction du diamètre nominal.

Le matériau constitutif des fils est, par exemple, un acier inoxydable, tel que celui référencé 316LVM. Il peut aussi être constitué par un super alliage, tel qu'un alliage de cobalt-chrome-nickel-molybdène-fer spécifié dans les normes ASTM F 1058 et ISO 5832-7, ou un alliage de Nickel et de Titane connu sous le nom de NITINOL.

Le diamètre extérieur d de la tresse 3 au repos est égal, au jeu fonctionnel près, à celui intérieur de la gaine textile 2 au repos, de manière que chacune d'elle puisse être déplacée longitudinalement par rapport à l'autre.

En pratique, l'implant tubulaire T, formé par la gaine 2 contenant une tresse 3, est choisi parmi une série d'implants se différenciant par leur diamètre intérieur, compris entre 8 et 14 millimètres avec incrémentation de 1,5 à 2 millimètres. Chacun des éléments de la série a une longueur de l'ordre de 30 centimètres, pouvant aller jusqu'à 50 centimètres dans certaines applications, et est réalisé dans des matériaux et dans des fils pouvant être sectionnés aux ciseaux.

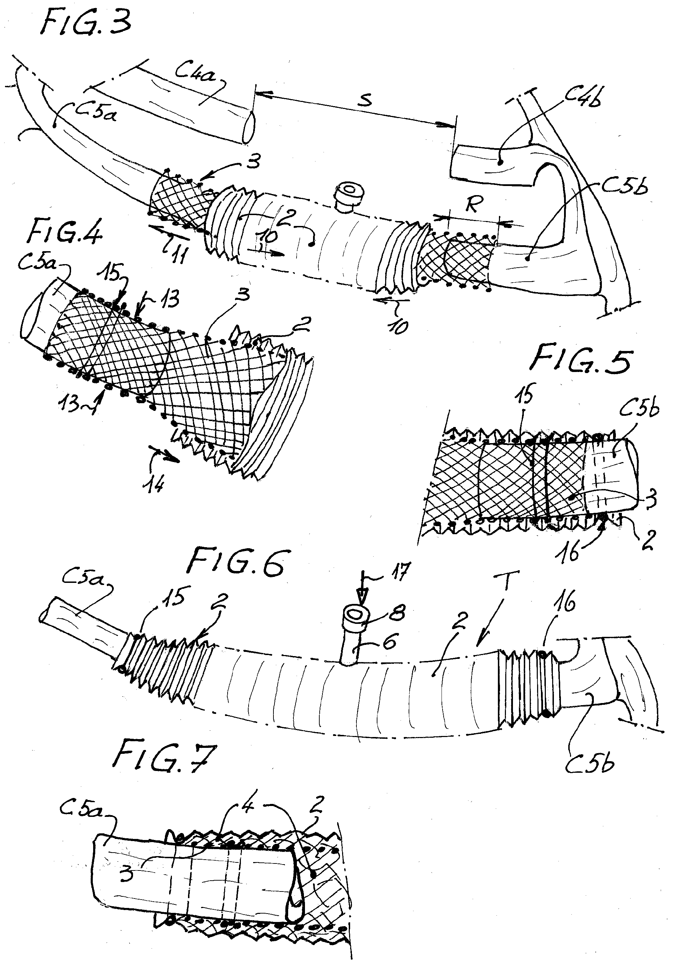

Pour construire une liaison intercostale entre les deux parties Cna et Cnb d'une énième côte Cn ayant fait l'objet d'une résection, comme montrée aux figures 2 et 3 pour la côte C5 fractionnée en C5a et C5b, le chirurgien mesure la distance S entre les deux parties C5a et C5b et détermine la longueur que doit avoir l'implant tubulaire T en prévoyant un recouvrement R des parties de côte sur une distance comprise entre 5 et 15 millimètres. Après avoir choisi un implant ayant un diamètre en rapport avec les dimensions et sections transversales des parties de côtes, le chirurgien coupe l'implant à la longueur nécessaire.

Ensuite et comme montré en détail par la flèche 10 à la figure 3, le chirurgien retrousse la gaine 2 sur chacune des extrémités de la tresse 3, pour en faciliter l'engagement sur chacune des parties de côte selon les flèches 11. Durant ce déplacement, le diamètre et la forme de la section transversale de la tresse 3 sont adaptés manuellement à ceux de la partie de côte C5a ou C5b.

En fin de mouvement d'engagement et alors que la tresse 3 est pincée manuellement selon les flèches 13 sur la partie de côte, par exemple C5a à la figure 4, son corps disposé hors de la côte est tiré dans le sens de la flèche 14 pour entraîner une réduction du diamètre de son extrémité sur la partie de côte et assurer son ancrage sur celle ci.

Bien que le serrage radial des extrémités de la tresse 3 sur les parties C5a et C5b de la côte suffise à assurer son positionnement, il peut parfois être nécessaire de verrouiller celui ci au moyen d'un fil de suture 15, visible à la figure 4.

Dans la phase suivante, et comme montré à la figure 5, les deux extrémités de la gaine 2, jusqu'alors retroussée sur la tresse, sont ramenées au dessus de ces extrémités, et même au delà, puis sont fixées par un fil de suture 16 ou par agrafage sur l'os.

Il peut alors être procédé à la suite de la reconstruction intercostale consistant, comme montré par la flèche 17 à la figure 6, à connecter l'embout 6 à une source de matériau de remplissage 4 et par exemple à un raccord ménagé à l'extrémité d'une seringue. Lors de l'injection, le matériau pénètre dans la cavité intérieure 2a de la gaine 2 et, grâce à la porosité de cette gaine, chasse vers l'extérieur l'air et les gaz contenus dans cette cavité.

La figure 7 montre que le matériau 4 remplissant la cavité 2a s'insère entre la gaine 2 et la partie C5a de côte, traverse la tresse 3 et, dans les zones extrêmes de cette tresse, forme un agent de liaison complémentaire de l'implant avec les parties C5a et C5b de la côte.

Pendant que le matériau de remplissage durci, ou polymérise, le chirurgien peut manuellement conformer l'implant afin qu'il prenne la forme la plus adaptée aux besoins de la reconstruction, des fonctions respiratoires et de la recherche d'un aspect esthétique.

Le matériau de remplissage est du type de ceux déjà utilisés en chirurgie réparatrice, par exemple pour la fixation des tiges de prothèses, et est constitué par un ciment de méthacrylate de méthyle.

Dans une forme de réalisation, ce ciment est un ciment phosphocalcique obtenu par mélange de deux phases :
- une phase pulvérulente composée d'un mélange de phosphate tétracalcique, de phosphate tricalcique et de glycérophosphate de calcium,
- et une phase liquide contenant de l'hydroxyde de calcium, de l'acide phosphorique et de l'eau.

Pour que la version basse densité s'injecte à la seringue, la phase solide du mélange contient du polydiméthyl siloxane.

Quand l'implantation est terminée, l'embout d'injection 6 est coupé au ras de la gaine 2, comme montré en C4 à la figure 8, sans qu'il soit nécessaire de l'obturer, puisqu'il l'est déjà par le matériau 4.

Dans l'implant ainsi obtenu, le matériau 4 est rigidifié par la tresse 3 qui se comporte alors comme une armature, de sorte que le dispositif présente une résistance au moins aussi grande que celle obtenue avec des implants à attelles métalliques, tout en s'ancrant aux côtes par des efforts périphériques, donc non localisés en quelques points, et moins dommageables pour l'os.

Ce mode de reconstruction de la cage thoracique peut s'appliquer aux côtes allant de la première à la dixième et aussi bien pour une réparation latérale, schématisée à la figure 2, que pour une réparation frontale, schématisée à la figure 8.

D'ailleurs à la figure 8, les références des composants de l'implant et de ses phases de mises en place sont les mêmes références que celles utilisées pour la description en référence aux figures 1 à 7.

## Revendications

1. Dispositif d'ostéosynthèse de la paroi thoracique composé d'une gaine tubulaire textile (2) implantable dans le corps humain, apte à contenir un matériau de remplissage (4) et dont les extrémités sont aptes à envelopper les extrémités (Cna et Cnb) de deux parties d'une même côte réséquée (Cn) caractérisé en ce cette gaine (2) :
- d'une part, contient une tresse (3), dont les fils biocompatibles (3a), métalliques ou polymériques, acceptent des déplacements relatifs les uns par rapports aux autres pour modifier localement le diamètre et la conformation de la tresse (3), afin que non seulement elle enveloppe mais aussi enserre l'extrémité d'une partie de côte (Cna ou Cnb),
- et, d'autre part, est solidaire d'un embout (6) d'injection d'un matériau biocompatible (4), assurant le remplissage de la gaine (2), le garnissage de la tresse (3) et la liaison par collage de la gaine (2) et de la tresse (3) avec les extrémités des parties (Cna et Cnb) de la côte (Cn).

2. Dispositif d'ostéosynthèse de la paroi thoracique selon la revendication 1 **caractérisé en ce que** la gaine tubulaire textile (2) présente une porosité laissant échapper l'air et les gaz lors de la coulée du matériau de remplissage (4) mais s'opposant à l'échappement de ce matériau.

3. Dispositif d'ostéosynthèse de la paroi thoracique selon la revendication 1 **caractérisée en ce que** la gaine tubulaire textile (2) est annelée par des annelures déformables permettant d'adapter sa conformation aux besoins de la reconstruction.

4. Dispositif d'ostéosynthèse de la paroi thoracique selon la revendication 1 **caractérisé en ce que** l'embout (6) d'injection est constitué par un corps tubulaire saillant à l'extérieur de la gaine textile (2) et dont une extrémité est liée à cette gaine par soudure ou couture (7), tandis que l'autre extrémité est munie d'un organe de raccordement (8) à des moyens d'injection du matériau (4) de remplissage et de renforcement.

5. Dispositif d'ostéosynthèse de la paroi thoracique selon la revendication 1, **caractérisé en ce que** la tresse (3), la gaine tubulaire (2) et l'embout (6) d'injection sont réalisés dans des matériaux sécables et avec des dimensions permettant de les sectionner avec des ciseaux.

6. Dispositif d'ostéosynthèse de la paroi thoracique selon les revendications 1 et 5 prises ensemble **caractérisé en ce que** :
- la tresse (3) est formée par des fils biocompatibles, métalliques (3a) ou en matière synthétique ou polymérique, ayant un diamètre extérieur compris entre 0,1 et 0,3 millimètres,
- tandis que la gaine tubulaire (2) est formée par tissage ou tricotage de fils en matériau biocompatible tels qu'en polyesters, polytétrafluoréthylène, polyéthylène ou propylène.

7. Dispositif d'ostéosynthèse de la paroi thoracique selon la revendication 1 **caractérisée en ce que** la tresse (3) a au repos un diamètre extérieur (d) égal, au jeu fonctionnel près, au diamètre intérieur (D) que la gaine (2) présente quand elle est au repos.

8. Dispositif d'ostéosynthèse de la paroi thoracique selon la revendication 1 **caractérisée en ce que** l'implant tubulaire T, comprenant la gaine (2) et la tresse (3), est choisi parmi une série d'implants se différenciant par leurs diamètres intérieurs compris entre 8 et 14 millimètres, avec incrémentation de 1,5 à 2 millimètres.

## Patentansprüche

1. Vorrichtung zur Osteosynthese der Brustkorbwand, bestehend aus einer in den menschlichen Körper implantierbaren rohrförmigen textilen Hülle (2), die imstande ist, ein Füllmaterial (4) zu enthalten und deren Enden imstande sind, die Enden (Cna und Cnb) von zwei Teilen einer selben resektierten Rippe (Cn) zu umhüllen,
**dadurch gekennzeichnet, dass** diese Hülle (2):
- zum einen ein Geflecht (3) enthält, dessen biologisch kompatible Metall- oder Polymerfäden (3a) relative Verlagerungen zueinander gestatten, um lokal den Durchmesser und die Ausbildung des Geflechts (3) zu verändern, damit es das Ende eines Rippenteils (Cna oder Cnb) nicht nur umhüllt, sondern auch umspannt,
- und zum anderen mit einer Einspritzspitze (6) eines biologisch kompatiblen Materials (4) fest verbunden ist, welches das Füllen der Hülle (2), die Ausfütterung des Geflechts (3) und die Verbindung durch Kleben der Hülle (2) und des Geflechts (3) mit den Enden der Teile (Cna und Cnb) der Rippe (Cn) sichert.

2. Vorrichtung zur Osteosynthese der Brustkorbwand nach Anspruch 1, **dadurch gekennzeichnet, dass** die rohrförmige textile Hülle (2) eine Porosität aufweist, die beim Fließen des Füllmaterials (4) die Luft und die Gase entweichen lässt, sich aber dem Entweichen dieses Materials entgegenstellt.

3. Vorrichtung zur Osteosynthese der Brustkorbwand nach Anspruch 1, **dadurch gekennzeichnet, dass** die rohrförmige textile Hülle (2) durch verformbare Ringelungen geringelt ist, die erlauben, ihre Ausbildung an die Bedürfnisse der Wiederherstellung anzupassen.

4. Vorrichtung zur Osteosynthese der Brustkorbwand nach Anspruch 1, **dadurch gekennzeichnet, dass** die Einspritzspitze (6) von einem rohrförmigen Körper gebildet ist, der aus der textilen Hülle (2) hervorsteht und von dem ein Ende mit dieser Hülle durch Schweißen oder Nähen (7) verbunden ist, wogegen das andere Ende mit einem Anschlussorgan (8) an Einspritzmittel des Füll- und Verstärkungsmaterials (4) verbunden ist.

5. Vorrichtung zur Osteosynthese der Brustkorbwand nach Anspruch 1, **dadurch gekennzeichnet, dass** das Geflecht (3), die rohrförmige Hülle (2) und die Einspritzspitze (6) aus teilbaren Materialien und mit Abmessungen hergestellt sind, die erlauben, sie mit einer Schere zu durchtrennen.

6. Vorrichtung zur Osteosynthese der Brustkorbwand nach den gemeinsam herangezogenen Ansprüchen 1 und 5, **dadurch gekennzeichnet, dass**:
- das Geflecht (3) von biologisch kompatiblen Metall-, Kunststoff- oder Polymerfäden (3a) mit einem Durchmesser zwischen 0,1 und 0,3 Millimeter inklusive gebildet ist,
- wogegen die rohrförmige Hülle (2) durch Weben oder Wirken von Fäden aus biologisch kompatiblem Material wie Polyestern, Polytetrafluorethylen, Polyethylen oder Propylen gebildet ist.

7. Vorrichtung zur Osteosynthese der Brustkorbwand nach Anspruch 1, **dadurch gekennzeichnet, dass** das Geflechts (3) in Ruhe einen Außendurchmesser (d) hat, der hinsichtlich seines Funktionsspiels nahezu dem Innendurchmesser (D) entspricht, den die Hülle (2) aufweist, wenn sie in Ruhe ist.

8. Vorrichtung zur Osteosynthese der Brustkorbwand nach Anspruch 1, **dadurch gekennzeichnet, dass** das rohrförmige Implantat T, das die Hülle (2) und das Geflecht (3) umfasst, aus einer Reihe von Implantaten ausgewählt ist, die sich durch ihre Innendurchmesser zwischen 8 und 14 Millimeter inklusive mit Inkrementierung von 1,5 bis 2 Millimeter unterscheiden.

## Claims

1. An osteosynthesis device of the chest wall consisting of a textile tubular sheath (2) which may be implanted in the human body, the capable of containing a filling material (4) and the ends of which are capable of enfolding the ends (Cna and Cnb) of two portions of a same resected rib (Cn) **characterized in that** this sheath (2):
- contains a braid (3) on the one hand, the biocompatible (3a), metal or polymeric threads of which accept relative displacements with respect to each other for locally modifying the diameter and the conformation of the braid (3), so that it not only enfolds but also clasps the end of a rib portion (Cna or Cnb),
- and, on the other hand, is secured to an endpiece (6) for injecting a biocompatible material (4), ensuring the filling of the sheath (2), the lining of the braid (3) and the adhesive bonding of the sheath (2) and of the braid (3) with the ends of the portions (Cna and Cnb) of the rib (Cn).

2. The osteosynthesis device of the chest wall according to claim 1, **characterized in that** the textile tubular sheath (2) has porosity allowing the air and the gases to escape upon casting the filling material (4) but opposing the escape of this material.

3. The osteosynthesis device of the chest wall according to claim 1, **characterized in that** the textile tubular sheath (2) is ringed with deformable wings allowing adaptation of its conformation to the needs of reconstruction.

4. The osteosynthesis device of the chest wall according to claim 1, **characterized in that** the injection endpiece (6) is formed by a tubular body protruding outside the textile sheath (2) and at one end of which is bound to this sheath by a weld or a seam (7), while the other end is provided with a member (8) for connecting to means for injecting the filling and reinforcing material (4).

5. The osteosynthesis device of the chest wall according to claim 1, **characterized in that** the braid (3), the tubular sheath (2) and the injection endpiece (6) are made in sectile materials and with dimensions allowing them to be severed with scissors.

6. The osteosynthesis device of the chest wall according to claims 1 and 5 taken together, **characterized in that**:
- the braid (3) is formed by biocompatible metal threads (3a) or in a synthetic or polymeric material, having an outer diameter comprised between 0.1 and 0.3 millimeters,
- while the tubular sheath (2) is formed by weaving or knitting threads in a biocompatible material such as in polyesters, polytetrafluoroethylene, polyethylene or propylene.

7. The osteosynthesis device of the chest wall according to claim 1, **characterized in that** the braid (3) has at rest an outer diameter (d) equal, to within the functional play, to the inner diameter (D) which the sheath (2) has, when it is at rest.

8. The osteosynthesis device of the chest wall according to claim 1, **characterized in that** the tubular implant T, comprising the sheath (2) and the braid (3), is selected from a series of implants which differ by their inner diameters comprised between 8 and 14 millimeters, with an incrementation of 1.5 to 2 millimeters.
